# EUROPEAN PATENT APPLICATION

(11) **EP 1 135 982 A1**
(43) Date of publication of application: **26.09.2001**
(21) Application number: 00957115.9
(22) Date of filing: 12.09.2000
(51) Int. Cl.: A01H 5/00, C12N 15/11, C12N 15/63, C12N 15/82

(54) **METHOD OF CONSTRUCTING MALE STERILE PLANT**

(30) Priority: 30.09.1999 JP 27930799
(71) Applicant: Japan Tobacco Inc., Tokyo 105-8422 (JP)
(72) Inventor: HAMADA, Kazuyuki, c/o Orynova K.K., Iwata-gun, Shizuoka 438-0802 (JP); NAKAKIDO, Fumio, c/o Orynova K.K., Iwata-gun, Shizuoka 438-0802 (JP)
(74) Representative: Reinhard - Skuhra - Weise & Partner
(86) International application number: JP0006222
(87) International publication number: WO0124616

(57) **Abstract**

A method for producing a male-sterile plant which shows normal morphology comparable to the intact plant but being male-sterile. More specifically, a method for producing a male-sterile plant characterized by comprising ligating a first promoter fragment to the upstream of an RNase gene, ligating a second promoter, which is the same as said first promoter or different therefrom, to the upstream of an RNase inhibitor protein gene, and transferring these genes into a plant genome to thereby make said plant substantially male-sterile.

## Description

The present application claims priority from Japanese Patent Application Hei 11-279307 filed on September 30, 1999, the disclosure of which is incorporated herein by reference.

### FIELD OF THE INVENTION

This invention relates to a method for producing a male-sterile plant by gene manipulation.

### PRIOR ART

Barnase is an RNase derived from *Bacillus amyloliquefaciens* (S. Nishimura and M. Nomura, Biochem. Biophys. Acta 30, 430-431:1958; R.W. Hartley, J. Mol. Biol., 202, 913-915:1988). It is an enzyme having 110 amino acid residues and hydrolyzing RNA. When expressed in cells, this enzyme inhibits the functions of the cells owing to its potent RNase activity and thus causes cell death in many cases. By using this characteristic, it is therefore expected that expressing the barnase gene in a specific site of a plant would result in the selective regulation of the function of that site.

PCT WO89/10396 discloses a technique whereby a male-sterile plant is obtained by constructing a male sterility gene by ligating the above-described barnase gene to the downstream of an anther tapetal cell-specific expression promoter and transferring the thus obtained gene into a plant. This male sterilization technique is highly useful in efficiently developing an F1 hybrid variety.

When a barnase gene is employed as a male sterility gene, however, it is frequently observed that the thus obtained male- sterile transgenic plant exhibits unfavorable characteristics. PCT WO96/26283 refers to this problem in rice. It is also reported that similar phenomena are observed in lettuce (Scientia Horticulturae 55, 125-139:1993; Arlette Reymaerts, Hilde Van de Wiele, Greta De Sutter, Jan Janssens: Engineered genes for fertility control and their application in hybrid seed production). According to this report, a plant with depressed activity was constructed by transferring a male sterility gene comprising a tobacco-origin anther-specific promoter (TA29) and barnase into lettuce.

Although the causes of these phenomena have not been accurately determined so far, it is assumed that the so-called "position effect" of the gene transfer site might affect the mechanism (PCT WO96/26283). More specifically, the aimed male-sterile plant can be constructed when the male sterility gene is expressed exclusively in the target site (i.e., anthers). However, there is a possibility that the barnase might be expressed, even though in an extremely small amount, in tissues other than anthers under the action of an expression regulator (for example, an endogenous enhancer) existing in the vicinity of the gene transfer site. In such a case, the unfavorable characteristics as described above might arise due to the potent enzymatic activity.

To overcome this problem, PCT WO96/26283 discloses a method using cauliflower mosaic virus 35S promoter (hereinafter referred to as CaMV35S promoter) which is expressed potently in tissues other than anthers. Namely, a Barstar, i.e., an inhibitory protein against barnase is employed therein. The Barstar gene ligated to the CaMV35S promoter is transferred into a plant simultaneously and then the Barstar gene is constitutively expressed in tissues other than anthers, thereby eliminating the effects of the barnase on tissues other than anthers.

In the male-sterile plant constructed by this method, however, the Barstar gene is expressed in the whole plant including leaves and albumen that is an edible part. It is not desirable from the viewpoint of the public acceptance of transgenic crops that the Barstar protein is expressed in parts where its expression is unnecessary.

### SUMMARY OF THE INVENTION

The present invention provides a method for producing a male-sterile plant that has normal morphology comparable to the intact species but being male-sterile.

The present invention provides a method for producing a male-sterile plant by expressing a foreign RNase in the anthers of the plant wherein the expression of the foreign RNase in tissues other than anthers is inhibited as much as possible to thereby construct a male-sterile plant showing normal morphology comparable to the original (i.e., intact) plant.

### BRIEF DESCRIPTION OF THE DRAWINGS

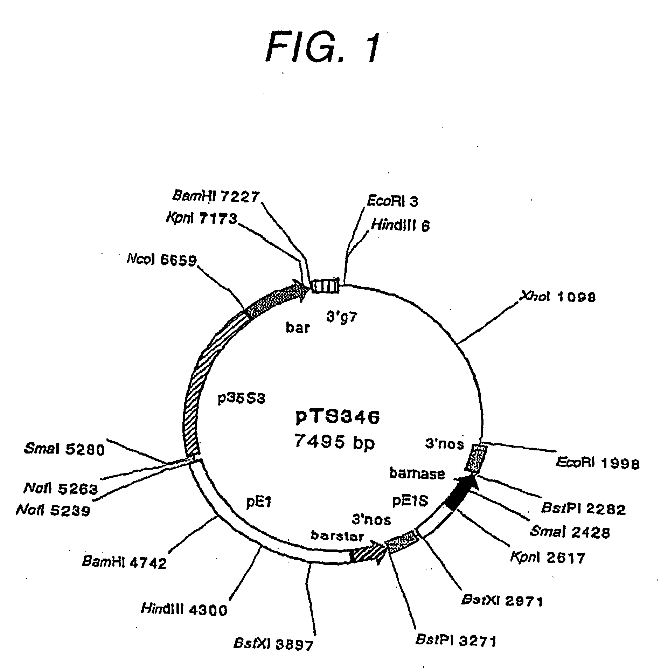

Fig. 1 is a model view which shows the structure of plasmid pTS346 for transforming plant cells which contains a Barstar gene under the control of an anther-specific E1 promoter and a barnase gene under the control of an anther-specific E1 promoter fragment in order from the 5'-end to the 3'-end.

### DETAILED DESCRIPTION OF THE INVENTION

The present inventors obtained two male sterile lines showing improvement in that they respectively exhibited a smaller degree of morphological abnormality from among rice male-sterile lines having been transfected with a genetic construct prepared by ligating a barnase gene to an anther-specific E1 promoter originating in rice. By analyzing these lines, it was found that the E1 promoter had been divided into two parts by an about 10 kb transposon originating in *Agrobacterium tumefaciens* LBA4404 employed in the transformation of rice. Based on this fact, it was assumed that the effect of the upper part of the E1 promoter on the expression of the barnase gene would have been compromised by the intervention of the transposon of 10 kb or more and, as a result, the expression of the barnase gene would have been depressed giving rise to the alleviation of morphological abnormality.

This finding indicated that the problem of the expression of an RNase in tissues other than anthers encountered in the conventional art would be overcome if an RNase gene (for example, a barnase gene) is ligated to the downstream of an anther-specific promoter which is made shorter than the full length (i.e., a promoter fragment) and the resultant construct is transferred into a plant genome. From this viewpoint, the present inventors further conducted intensive studies and thus produced a transgenic rice plant by transferring a Barstar gene ligated to the downstream of a rice anther-specific E1 promoter simultaneously with an RNase gene ligated to a fragment of an anther-specific promoter fragment, thereby achieved further improvement in the morphology of male-sterile rice lines.

Accordingly, the present invention provides a method for producing a male-sterile plant characterized by comprising ligating a first promoter fragment to the upstream of an RNase gene, further ligating a second promoter, which is the same as the first promoter or different therefrom, to the upstream of an RNase inhibitor protein gene, and transferring these genes into a plant genome to thereby make the plant substantially male-sterile.

It is preferable that the RNase gene to be used in the present invention is barnase gene. Barnase gene may be employed as such. Alternatively, it may be naturally or artificially mutated so as to modify the activity of the enzyme encoded thereby or the tissue specificity thereof. For the purpose of the present invention, use can be made of the gene of any enzyme which can act as an RNase in plant cells (in particular, anthers) and thus inhibit protein biosynthesis in the cells. Examples of such enzymes include pancreas RNase A, RNase T1 from *Aspergillus oryzae* and Sarnase originating in *Streptomyces aureofaciens* (European Patent Publication No. 053799).

The promoter to be ligated to the upstream of the RNase gene (i.e., the first promoter) can be any promoter, which can induce the expression of the RNase gene to be employed in plant cells. As examples, tobacco TA29 promoter (J. Seurinck et al., Nuc. Acid. Res., 18, 3403, 1990) and *Arabidopsis* A9 promoter (Wyatt Paul et al., Plant Mol. Biol., 19, 611-622, 1992) may be mentioned. It is preferable that the first promoter is a promoter specific to the anther of the plant which is to be made male-sterile. In the case of rice, there are known anther-specific promoters such as E1 promoter, T72 promoter, T42 promoter (each described in PCT WO92/13956), Osg6B promoter, Osg4B promoter (each reported by T. Tsuchiya et al., Plant Mol. Biol. 26, 1737-1746, 1994) and the like. The sequence of the E1 promoter is represented by SEQ ID NO:6.

It is essential to use the first promoter as a fragment. The term "fragment" as used herein in connection with the first promoter means a sequence that at least partly differs from the promoter sequence occurring in nature. For example, the fragment is a promoter having deletion, substitution or insertion in the 3'-region, the internal region and/or the 5'-region including a case of being divided into upstream and downstream regions with an intervening sequence. It is preferable that, as a result of the deletion, substitution or insertion, the fragment has up to about 50% homology, still preferably up to about 70% homology and particularly preferably up to about 90% homology, with the natural promoter sequence. As one of the preferable examples of the first promoter fragments, the fragment originating in the E1 promoter as represented by SEQ ID NO:7 may be mentioned. Also, it is preferable to use, as the first promoter fragment, a sequence obtained by modifying the above-described sequence by substitution, deletion or insertion of one or more nucleotides, so long as it has the same promoter activity as the fragment represented by SEQ ID NO:7. The expression "to modify by substitution, deletion or insertion of nucleotides" as used herein means such a modification as is available by subjecting, for example, ten or less (preferably five or less) nucleotides to artificial manipulation such as site-specific mutagenesis.

The first promoter fragment is ligated to the upstream of the RNase gene in a functional manner. The term "functional" means that the promoter fragment is ligated to such a position as allowing inducing the expression of the RNase gene.

It is also essential in the present invention to use an RNase inhibitor protein gene. This gene may be appropriately selected depending on the RNase gene employed. In the case where the RNase gene is barnase gene, for example, it is preferable to use Barstar gene as the RNase inhibitor protein gene. A second promoter is ligated in a functional manner to the upstream of the RNase inhibitor protein gene. The second promoter, which induces the expression of the RNase inhibitor protein gene to be employed in plant cells, may be either the same as the first promoter or different therefrom. In case where the second promoter is the same as the first promoter, it is not always necessary to employ the second promoter in the form of a fragment.

The RNase gene, which has been ligated to the first promoter fragment in a functional manner, may be located either downstream or upstream of the RNase inhibitor protein gene which has been ligated to the second promoter in a functional manner. Also, the distance between these genes is not particularly limited.

The plant that can be made male-sterile by the method of the present invention is not particularly limited. As examples thereof, rice, corn, tobacco, lettuce and rapeseed may be mentioned. Among all, rice and corn are particularly preferable.

To transfer the genes into a plant genome to make the plant male-sterile in the method of the present invention, use may be made of the Agrobacterium method, the electroporation method, the particle gun method, etc. It is preferable to use the Agrobacterium method. Details of the procedure may be appropriately determined by a person skilled in the art by reference to, for example, the method described in PCT WO92/13957. For example, the genes can be transferred into a plant cell genome by the following steps:
(1) providing a first promoter fragment;
(2) prividing an RNase gene;
(3) ligating the RNase gene to the downstream of the first promoter fragment in a functional manner;
(4) providing a second promoter;
(5) providing an RNase inhibitor protein gene;
(6) ligating the RNase inhibitor protein gene to the downstream of the second promoter in a functional manner;
(7) integrating the RNase gene, which has been ligated to the downstream of the first promoter fragment, and the RNase inhibitor protein gene, which has been ligated to the downstream of the second promoter, into T-DNA with the use of restriction enzyme sites;
(8) inserting the T-DNA from step (7) into a Ti plasmid;
(9) amplifying the Ti plasmid from step (8) in an agrobacterium host cells, if needed; and
(10) infecting plant cells with the agrobacterium from step (9) in order to transfer the RNase gene, which has been ligated to the downstream of the first promoter fragment, and the RNase inhibitor protein gene, which has been ligated to the downstream of the second promoter, into the genome of the plant cells.

The transgenic plant cells thus obtained can be regenerated into an individual plant starting with callus culture by using the method described in, for example, Y. Hiei et al., Plant J. 6, 271-282:1994.

In the above-described process, the first promoter fragment may be prepared by cleaving the first promoter with appropriate restriction enzymes. Alternatively, it may be prepared by amplifying the sequence, which is obtained by deleting unnecessary regions from a plasmid containing the first promoter and the RNase gene, by PCR with the use of appropriate primers, as will be described in Example hereinafter. Also, it is to be understood that various changes in the order of the above steps (in particular, steps (1) to (7)) or materials employed therein fall within the scope of the present invention. Further, it is possible to transform plant cells by using Ti plasmids containing different T-DNAs, of which one carrying the RNase gene which has been ligated to the downstream of the first promoter fragment, while the other carrying the RNase inhibitor protein gene which has been ligated to the downstream of the second promoter. Furthermore, calluses having the transferred genes can be easily selected by preliminarily providing a selection marker in the T-DNA of the step (7). Examples of such a marker include Bar gene employed in the following Example and a hygromycin resistant (HPT) gene. However, it is not always necessary to use a selection marker. The achievement of transformation may be judged based on the fact that the plants regenerated from calluses of transgenic cells have been made male-sterile.

The present invention further provides a vector for transferring the above genes into a plant cell genome by the agrobacterium method as described above. This is a vector which has a T-DNA containing i) a promoter fragment and an RNase gene expressible under the control of said promoter, and ii) a promoter being the same as the above-described promoter or different therefrom and an RNase inhibitor protein gene expressible under the control of said second promoter, so that the vector can transfer the T-DNA into a plant cell genome when placed in an agrobacterium-infected plant cells. It is preferable that the vector of the present invention is constructed by integrating the T-DNA containing the above-described genetic elements i) and ii) into Ti plasmid for transforming plants. Various Ti plasmids for transforming plants are known and available at present. For example, pAL4404 carried by Agrobacterium tumefaciens strain LBA4404 can be mentioned. The vector according to the invention may contain, if necessary, one or more elements selected from among a replication origin for amplification in an agrobacterium, terminators located respectively downstream of the RNase gene and the RNase inhibitor protein gene and/or a marker gene appropriate for selecting transgenic plant cells.

The present invention furthermore provides a transgenic plant cell which contains i) a promoter fragment and an RNase gene expressible under the control of said promoter, and ii) a promoter being the same as the above-described promoter or different therefrom and an RNase inhibitor protein gene expressible under the control of said promoter, having been transferred into the genome, and a male-sterile plant regenerated from said transgenic cell.

### EFFECT OF THE INVENTION

A male-sterile plant, which exhibits less or no abnormalities in morphology or flowering properties compared with male-sterile plants made by the conventional constructs, can be produced by transferring a barnase gene ligated under a promoter having a reduced activity due to the deletion of an upstream region together with a Barstar gene ligated under a rice anther-specific promoter, and introducing these genes into the genome of a plant cell.

Now, the present invention will be illustrated in greater detail with reference to the following Example. However, it is to be understood that the invention is not to be construed as being restricted thereto.

### Example

As a template for amplifying a barnase gene ligated to the downstream of an E1 promoter fragment, use was made of plasmid pTS172 (Japanese Patent Application Hei 10-220060). After amplifying by the PCR method with the use of primers 172del-F (SEQ ID NO:1) and 172del-R (SEQ ID NO:2) each having a PstI cleavage site, the obtained product was treated with PstI. Thus, a fragment of E1 promoter from pTS172 was obtained. The fragment had a cohesive end at each side and comprised exclusively of about a 360 bp sequence including the initiation point and its upstream of the E1 promoter. The both ends of this fragment were ligated together to cyclize it again by using T4 DNA ligase in a conventional manner, thus giving a novel plasmid pTS172Δ (SEQ ID NO:3). That is to say, in the plasmid pTS172Δ, the E1 promoter region (about 1.7 kb) of the original plasmid pTS172 was substituted by the 356 bp fragment represented by SEQ ID NO:7.

Next, a fragment (SEQ ID NO:4) comprising an E1 promoter (PCT WO92/13956) ligated to a Barstar gene (R.W. Hartley, J. Mol. Biol. 202, 913-915:1988) was blunt-ended and integrated into the PstI site of pTS172Δ to give a plasmid pTS346 (SEQ ID NO;5). Fig. 1 illustrates the structure of pTS346.

A 5.5kb fragment was excised from pTS346 with EcoRI and then inserted into the EcoRI site of pSB11BS (the structure of which will be described just below). Further, the T-DNA region was integrated into an acceptor vector pSB1 (T. Komari et al., Plant J. 10, 165-174:1996) by way of homologous recombination. The resulted recombinant plasmid (pSB1346) was introduced into *Agrobacterium tumefaciens* LBA4404, which was used in transforming rice (variety: Asanohikari). The plasmid pSB11BS was constructed by integrating the Barstar gene (R.W. Hartley, J. Mol. Biol. 202, 913-915:1988) into the StuI site of intermediate vector pSB11 (T. Komari, Plant J. 10, 165-174:1996).

By this *Agrobacterium tumefaciens* LBA4404 having the recombinant plasmid, rice (variety: Asanohikari) was transformed. Although the transformation was performed basically in accordance with the method of Hiei et al. (Plant J. 6, 271-282:1994), phosphinothricin (concentration: 10 mg/L) was employed for screening transgenic plants since the male sterility-gene construct contained a bar gene encoding phosphinothricin acetyl transferase as a selection marker.

A comparison with the construct pSB1172 (refer to PCT/JP99/04167 filed on August 3, 1999) comprising an E1 promoter of the normal length but lacking any Barstar gene indicated that the transformation efficiency and the ratio of transgenic plants with normal morphology were remarkably improved as shown in the following table.

**Table 1:**

| Transformation efficiency | | | | |
|---|---|---|---|---|
| | No. of infected calluses | No. of re-differentiated calluses | No. of PCR-positive lines | No. of morphologically normal male sterile lines |
| pSB1346 | 681 | 45 | 37/38* | 15/37 (40.5%) |
| pSB1172 (control) | 2838 2838 | 83 83 | 52/83 52/83 | 9/52(17.3%) 9/52(17.3%) |

| | | | | |
|---|---|---|---|---|
| *: examined 38 lines among 45. | | | | |

## Claims

1. A method for producing a male-sterile plant comprising ligating a first promoter fragment to the upstream of an RNase gene, ligating a second promoter, which is the same as said first promoter or different therefrom, to the upstream of an RNase inhibitor protein gene, and transferring these genes into a plant genome to thereby make said plant substantially male-sterile.

2. The method for producing a male-sterile plant as claimed in claim 1 wherein said RNase gene is barnase gene.

3. A method for producing a male-sterile plant as claimed in claim 1 or 2 wherein said RNase inhibitor protein gene is Barstar gene.

4. A method for producing a male-sterile plant as claimed in any of claims 1 to 3 wherein the first promoter and the second promoter are both promoters causing anther-specific expression.

5. A method for producing a male-sterile plant as claimed in any of claims 1 to 4 wherein said first promoter is a part of the promoter represented by SEQ ID NO:6.

6. A method for producing a male-sterile plant as claimed in any of claims 1 to 5 wherein said first promoter is the promoter represented by SEQ ID NO:7.

7. A promoter comprising a part of the sequence represented by SEQ ID NO:6.

8. A promoter comprising the sequence represented by SEQ ID NO:7 or a sequence obtained by modifying the same by the substitution, deletion or addition of one or more nucleotides.

9. A plasmid vector which has a T-DNA containing i) a first promoter fragment and an RNase gene the expression of which is induced by the first promoter, and ii) a second promoter being the same as the first promoter or different therefrom and an RNase inhibitor protein gene the expression of which is induced by the second promoter, said plasmid vector being capable of introducing said T-DNA into a plant cell genome when placed in agrobacterium-infected plant cells.

10. A transgenic plant cell which contains i) a first promoter fragment and an RNase gene the expression of which is induced by the first promoter, and ii) a second promoter being the same as the first promoter or different therefrom and an RNase inhibitor protein gene the expression of which is induced by this second promoter transferred into the genome thereof, and a male-sterile plant regenerated from said cell.
